# EUROPEAN PATENT SPECIFICATION

(11) **EP 3 952 958 B1**
(45) Date of publication and mention of the grant of the patent: **04.11.2026**
(21) Application number: 20787601.2
(22) Date of filing: 09.04.2020
(51) Int. Cl.: A61M 5/315, A61M 5/168, A61M 5/178, A61M 5/28, A61M 5/142

(54) **INJECTION DEVICE**
INJEKTIONSVORRICHTUNG
DISPOSITIF D'INJECTION

(30) Priority: 09.04.2019 US 201962831487 P
(43) Date of publication of application: 16.02.2022
(73) Proprietor: Congruence Medical Solutions, LLC, Hanover, MD 21079-1997 (US)
(72) Inventor: CASTAGNA, Lou, Middletown, Pennsylvania 17057 (US); WETZEL, Lance, York, Pennsylvania 17407 (US); SHETTY, Gautam Nithyanand, Hanover, Maryland 21076 (US)
(74) Representative: Boult Wade Tennant LLP
(86) International application number: PCT/US2020/027478
(87) International publication number: WO 2020/210498

(56) References cited:
- WO-A1-2013/114357
- WO-A1-2015/117131
- WO-A1-2018/125829
- WO-A1-2019/016351
- WO-A2-2010/033770
- US-A- 5 496 293
- US-A1- 2012 203 184
- US-A1- 2013 150 803
- US-A1- 2014 257 197
- US-A1- 2014 343 511

## Description

### TECHNICAL FIELD

This patent disclosure relates generally to injection devices and, more particularly to multi-dosing injection devices for sequentially delivering several fractions of a total volume of injectable fluid available in a syringe.

### BACKGROUND

There are a number of current and emerging clinical applications that require delivering at only a fraction of the total available injectable fluid volume using a syringe a time. These dose volume fractions can range from microliters through milliliters. These include applications in disciplines such as ophthalmology, oncology, dental, dermatology, nephrology, vaccines, rheumatology, etc. Administration of injections may be required at multiple locations within an organ or tumor. For example, in case of skin carcinomas, injections may be required in multiple lesions to be treated.

The most common approach to splitting the total deliverable volume into multiple doses is by using injection volume gradations on the syringe as a reference. For example, to split a 1 mL drug solution in a syringe into 10 fractions of 0.1 mL each, the clinician administering the drug solution can achieve 10 injections of 0.1 mL by controlling the start of injection position and end of injection position. The difference between the aforementioned positions yields the volume to be injected.

The cognitive burden placed on the clinician includes calculation of injection dose volume for each injected dose volume and memorization of start of dose and end of dose positions for each injected volume. Simultaneously, clinician is required to ensure that the dose is injected in the correct location. Cognitive burdens associated with conventional injection systems could pose potential dexterity challenges with injection procedure, which may diminish effectiveness of injected therapy or cause potential harm. This problem becomes particularly acute for injectable drugs with a narrow therapeutic window or investigational treatments where treatment efficacy is to be determined (or unknown).

In order to minimize the cognitive burden with using a single conventional syringe for multiple doses, a clinician could have multiple syringes filled with therapeutic agent prefilled and ready to inject only the desired amount. There is inherent inefficiency and waste in this approach since there is drug priming required for each of the syringes, and hence increase cost of treatment. Also, the procedure would now involve multiple personnel to refresh and replace the syringes being used. This additional handling could also pose exposure risks to clinical personnel in case of potent treatments involving virus, immunotherapeutic agents, chemotherapies, etc.

The delivery conduit is typically an injection needle (or rigid delivery cannula), a catheter or a luer lock access site. Any delivery system is first primed to ensure that all of the air in the delivery conduit is purged. In case of applications requiring splitting the dose into equal fractions of the total available dose in the syringe, priming is done only once prior to delivery of the first fractional dose.

In addition to using dose markings, various device-based approaches have been proposed to split the total available volume in the syringe. These device-based approaches typically require the stereotactic reference to change with administration of each fraction of the dose; this would require the clinician to adjust their grip with injection of each fraction. While it is possible it maintain the same differential between start and end of dose positions, the absolute positions of the start and end of dose (stereotactic reference) is constantly changing with delivery of each dose fraction. This potential lack of stability can be particularly problematic in applications involving injections in sensitive organs, and in applications such as in cosmetic dermatology, where any disturbance during injection when using a needle can cause a cosmetic defect and/or injury. This issue of constantly changing stereotactic reference can also pose an extra challenge and complexity in incorporating such a device with a robotic delivery system.

Many device-based approaches also require an extra step from the user to transition from delivery of one fractional dose to another fractional dose. While this can be accommodated in some applications, other applications, such as injections in sub-retinal space in the eye, require minimizing number of steps involved. Device designs that do not require an additional step to deliver the next fractional dose would also be more compatible with a robotic delivery system.

Some applications require administration into a high pressure line, such as injection in a blood vessel. This requires that there be no ingress of blood back into the syringe. This is now achieved by clinician maintaining pressure on a syringe plunger rod. If pressure is not maintained, there is potential of a blood spill when the plunger rod is pushed back to the non-patient end, and hence risk of blood exposure to the healthcare professional.

Some other applications require accurate, precise delivery of sub-milliliter fractional doses. This can be particularly challenging without a device to split the dose from a syringe, which is typically used to deliver milliliter drug dose volumes into equal microliter fractions. Even within microliter delivery, the problem with delivering an accurate, precise dose becomes more acute with fractional doses of 100 microliters or less. Accuracy and precision of delivered fractional dose is also critical in applications involving injection of potent drugs that have a very narrow therapeutic window or drugs that may be harmful effects outside of the target delivery area.

While some multidosage devices have been proposed, these often include complicated structures that may be prone to damage or improper operation, and may be difficult or expensive to manufacture. Still other devices may not provide reliable and convenient operation in that the actuation mechanisms may not be in line with the actual administration needle.

U.S. Publication 2014/0257197 A1 discloses a multiple dosage injector that includes a rack and pinion dosage mechanism that includes a ram, a ratchet gear, a crank arm and a user-manipulable push button. The crank arm includes a pawl tooth and pivots about a pivot point. A crank arm engagement member slideably engages with the push button slot such that movement of the push button causes the crank arm engagement member to move along the push button slot, causing rotation of the crank arm about the pivot point.

International Publication WO 2013/114357 A1 discloses a cradle for a syringe wherein depression of a button at the end of the cradle causing a stepped advancement of the plunger of the syringe. The actuation mechanism is offset from the syringe such that movement of the button and attached rod is transferred to the syringe plunger by a cantilevered arrangement.

International Publication WO 2015/117131 A1 discloses a device that includes expanding plunger rods which enable smaller packaging footprints. While the plunger rod may be telescoped from a compact, shipping configuration to an extended use structure, the plunger rod is not designed for multiple measured administrations.

International Publication WO 2010/033770 A2 discloses a medical injector with a ratching plunger having a plunger rod with ratchet teeth attached to a stopper of a reservoir. The injector further includes a cylindrical actuator having pawls that engage the ratchet teeth to provide movement of the ratcheting plunger in a distal direction for administration, and a cylindrical indexer having pawls that engage the ratchet teeth to prevent movement of the ratcheting plunger in a proximal direction.

International Publication WO 2019/016351 A1 discloses an autoinjector that includes a blocking system blocking the piston rod in the proximal rest position. Once the blocking system is released and the device is activated, the fluid from an attached syringe is automatically delivered. The autoinjector device is not utilized for subsequent injections from the same coupled syringe.

### SUMMARY

The disclosure describes, in one aspect, a controlled multidosing delivery device for use with a syringe including a barrel, plunger stopper, and a delivery conduit. The controlled multidosing delivery device includes a housing, a plunger rod and a drive shell. The housing defines an axis and including a proximal end, a distal end, an axially extending chamber including a first opening to the proximal end of the housing and a second opening to the distal end of the housing. The distal end of the housing is adapted for attachment to the syringe barrel along the axis such that the housing and syringe barrel are coaxial. The plunger rod includes an elongated shaft having a proximal end and a distal end. A contact button is disposed at the proximal end of the elongated shaft, and a pushing feature is disposed at the distal end of the elongated shaft. A portion of the elongated shaft is disposed within the axially extending chamber of the housing. The proximal end of the elongated shaft extends at least partially from the proximal end of the housing with the contact button being disposed external to the housing. A biasing structure is disposed to bias the contact button away from the housing. A retaining structure is adapted to inhibit removal of the plunger rod through the first opening of the housing and permit the plunger rod to translate axially a predetermined distance. The drive shell is disposed within the axially extending chamber of the housing. The drive shell includes a head disposed for translation along the axis to confront the stopper plunger of the syringe. The drive shell further includes an elongated drive arm and an elongated retention arm. The elongated drive arm includes a plurality of engagement surfaces. The plunger rod is disposed between the cclongated drive arm and the elongated retention arm with the pushing feature of the plunger rod biased toward at least one of the engagement surfaces. The pushing feature is disposed to engage the at least one of the engagement surfaces to translate the drive shell in a distal direction. A retention feature includes at least one retention finger and a plurality of retention surfaces. The retention feature is adapted to inhibit proximal movement of the drive shell within the axially extending chamber when retention finger is engaged with at least one of the plurality of retention surfaces. At least one of the at least one retention finger and the plurality of retention surfaces is associated with the elongated retention arm of the drive shell; the other of the retention finger and the plurality of retention surfaces is associated with the housing. Depression of the contact button axially translates the elongated shaft and pushing feature in engagement with the at least one of the engagement surfaces in a distal direction along the axis to translate the drive shell in the distal direction based upon the predetermined distance to cause corresponding movement of the plunger stopper within the barrel for a dose delivery, whereby the at least one retention finger engages with at least one of the plurality of retention surfaces to maintain an axial position of the drive shell in the housing following movement of the drive shell in the distal direction, and whereby the biasing structure translates the plunger rod in the proximal direction following translation of the drive shell.

The disclosure further describes, in another aspect, a method of assembling the controlled multidosing delivery device including inserting the drive shell into the axially extending chamber in the housing, inserting the distal end of the plunger rod into the housing to position the contact button for depression, and coupling a retaining structure with the plunger rod to prevent removal of the plunger rod from the housing.

In yet another aspect, the disclosure describes various applications of the disclosed device.

### BRIEF DESCRIPTION OF THE DRAWING(S)

FIG. 1 is an exploded isometric view of an exemplary controlled multidosing delivery device and syringe according to this disclosure.
FIG. 2A is a side elevational view of a housing of the exemplary controlled multidosing delivery device of FIG. 1.
FIG. 2B is a bottom view of the housing of FIG. 2A.
FIG. 2C is a top view of the housing of FIGS. 2A-2B.
FIG. 2D is an isometric view of the housing FIGS. 2A-2C from a generally top position.
FIG. 2E is an isometric bottom view of the housing of FIGS. 2A-2D from a generally bottom position.
FIG. 3A is an isometric view of a clip of the exemplary controlled multidosing delivery device of FIG. 1.
FIG. 3B is a top view of the clip of FIG. 3A.
FIG. 3C is a side elevational view of the clip of FIGS. 3A-3B.
FIG. 3D is a front elevational view of the clip of FIGS. 3A-3C.
FIG. 4 is a fragmentary isometric view of a proximal end of a syringe and clips being assembled into a distal end of the exemplary controlled multidosing delivery device of FIG. 1.
FIG. 5 is an isometric view of a syringe being assembled into the distal end of the exemplary controlled multidosing delivery device of FIG. 1 utilizing an exemplary wrench tool, the wrench tool being shown in fragmentary form.
FIG. 6A is an isometric view of a plunger rod of the exemplary controlled multidosing delivery device of FIG. 1.
FIG. 6B is a side elevational view of the plunger rod of FIG. 6A.
FIG. 7A is a front elevational view of a drive shell of the exemplary controlled multidosing delivery device of FIG. 1.
FIG. 7B is a front and side elevational view of the drive shell of FIG. 7A.
FIG. 8 is a cross-sectional of an assembled exemplary controlled multidosing delivery device of FIG. 1.
FIG. 9A is an isometric view of a drive shell being assembled to a housing to construct the exemplary controlled multidosing delivery device of FIG. 1.
FIG. 9B is a side view of the drive shell and the housing of FIG. 9 in an assembled position.
FIGS. 10 and 11 illustrate the operation the exemplary controlled multidosing delivery device of FIG. 1 in a sequence of deliveries of an injectable fluid.
FIG. 12A is a side elevational view of an alternative embodiment of an exemplary controlled multidosing delivery device.
FIG. 12B is a side elevational view of a cover of the exemplary controlled multidosing delivery device of FIG. 12A.
FIGS. 12C-12E are alternative embodiments of a drive shell that may be incorporated with the exemplary controlled multidosing delivery device of FIG. 12A.
FIG. 13 illustrates the operation of the exemplary controlled multidosing delivery device of FIG. 12A in a sequence of deliveries of an injectable fluid.
FIG. 14 is a side elevational view of a drive shell and retaining arrangement of an alternate embodiment an exemplary controlled multidosing delivery device.
FIG. 15 is a schematic view of an exemplary application of a controlled multidosing delivery device according to this disclosure
FIG. 16 is a schematic view of an alternative exemplary application of a controlled multidosing delivery device according to this disclosure

### DETAILED DESCRIPTION

This disclosure relates to a controlled multidosing delivery device utilized to sequentially delivering several fractions of the total available injectable fluid available in a syringe; these injection fraction volumes may be equal or unequal. For the purposes of this disclosure, the term "injectable fluid" includes any injectable fluid, including, but not limited to therapeutic agent, injectable substance, drug solution, stem cells, etc., and vice versa, unless otherwise apparent from the context. For the purposes of this disclosure, the term "delivery conduit" is a structure through which an injectable fluid may be delivered, including, but not limited to, a cannula, a needle, catheter, an elongated tubular structure, etc., and vice versa, unless otherwise apparent from the context. Also for the purposes of this disclosure, the terms "user" and "clinician" and "operator" are used interchangeably and include any individual or individuals operating the device unless otherwise apparent from the context.

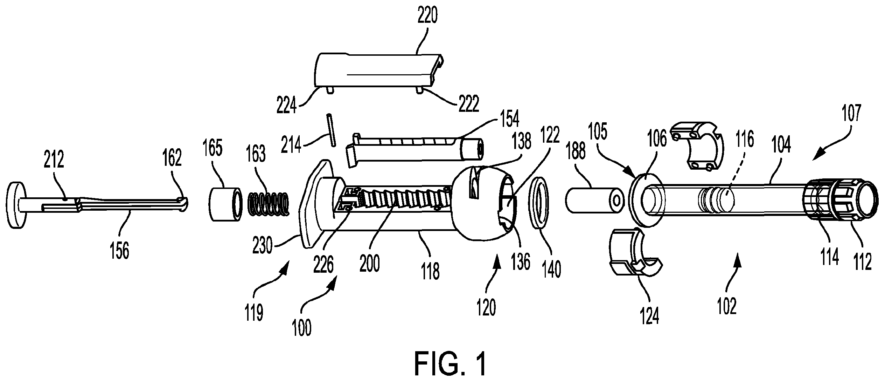

Turning to FIG. 1, there is illustrated an exploded isometric view of a controlled multidosing delivery device 100 according to the disclosure in conjunction with a syringe 102. For the purposes of this disclosure, the term "proximal" will be used to identify the portion or end of an associated structure that is disposed toward the user or operator of the controlled multidosing delivery device 100 and syringe 102, while the term "distal" will be used to identify the portion or end of an associated structure that is disposed away from the user or operator of the controlled multidosing delivery device 100 and syringe 102

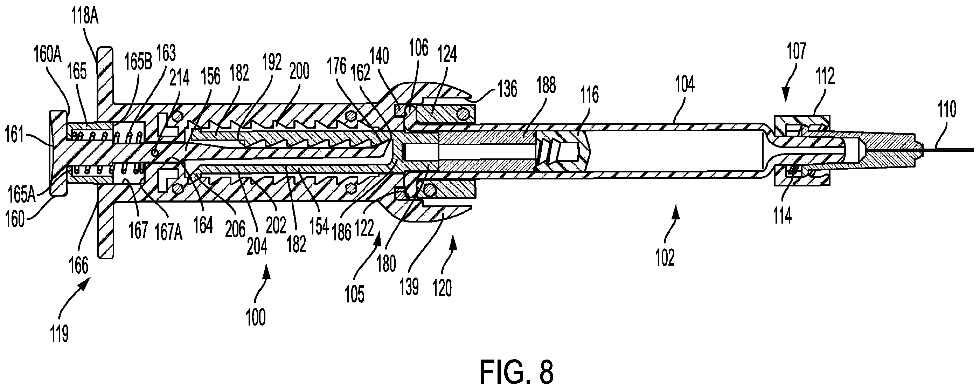

Referring to the cross-section of FIG. 8 in addition to FIG. 1, the syringe 102 includes a barrel 104 having a proximal end 105 including a flange 106 and a distal end 107 for attachment to a delivery conduit 108. The delivery conduit 108 may be, for example, a catheter or an injection needle, such as the illustrated injection needle 110. While the delivery conduit 108 may be coupled to the barrel 104 by any appropriate arrangement, in at least one embodiment, the delivery conduit 108 is attached by a luer lock adapter 114 with tip cap 112. It will be appreciated that an alternate attachment mechanism may be provided, and that the term "luer lock" is used in a generic sense and is intended to include other attachment mechanisms. The barrel 104 is adapted to receive an injectable fluid. In order to maintain the injectable fluid within the barrel 104, a plunger stopper 116 is disposed within the barrel 104. The plunger stopper 116 is adapted to move axially within the barrel 104 to dispense an injectable fluid contained therein via the delivery conduit 108.

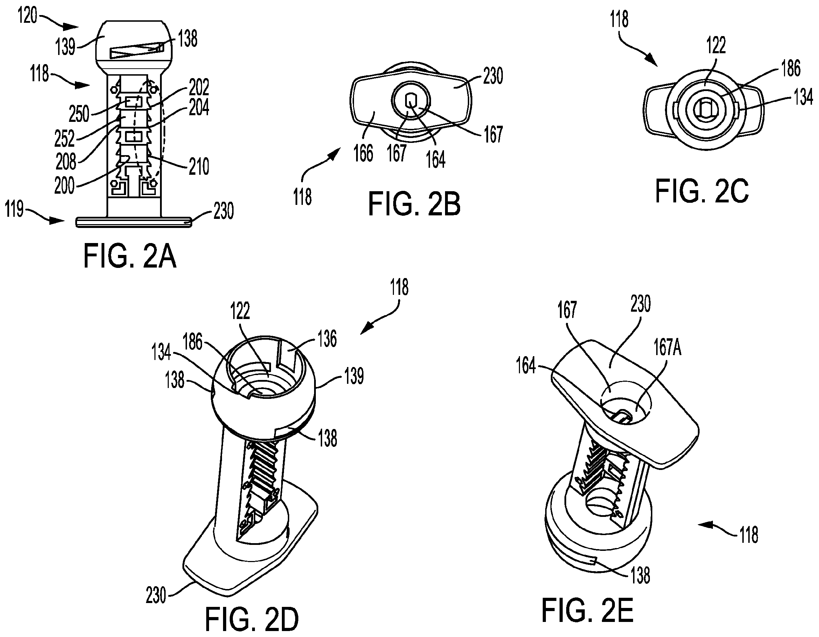

The controlled multidosing delivery device 100 is provided for attachment to the syringe 102. The controlled multidosing delivery device 100 includes a housing 118, which includes a proximal end 119 and a distal end 120. The distal end 120 of the housing 118 is adapted for coupling to the proximal end 105 of the syringe 102. The housing is illustrated in greater detail in FIGS. 2A-2E. In order to couple the housing 118 to the syringe 102, the distal end 120 is provided with a channel 122, as seen most clearly in FIG. 2D. In this embodiment, the channel 122 is axially extending, facilitating an axial sliding of the flange 106 of the syringe 102 with the distal end 120 of the housing 118.

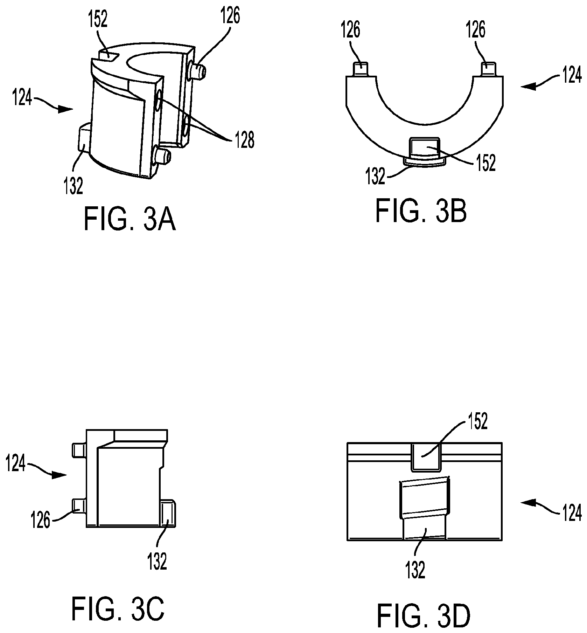

In order to couple the flange 106 with the housing 118, one or more clips 124 are provided. As seen most clearly in FIG. 1, the illustrated embodiment includes two clips 124 of generally arcuate structures. To facilitate accurate registration of the clips 124 together with the proximal end 105 of the barrel 104 and the flange 106, the clips 124 may include mating protrusions 126 and cavities 128 (see FIGS. 3A-3C). The protrusions 126 are sized for receipt into the cavities 128 in order to couple the clips 124 to the proximal end 105 of the syringe 102. While the protrusions 126 and cavities 128 of the illustrated embodiment are annular structures, it will be appreciated that the protrusions 126 and cavities 128 may be of an alternate design or structure. Further, while the illustrated clips 124 are of the same or similar structure, it will be appreciated that the one or more clips may be of any appropriate design adapted for attachment to the syringe 102 and to the housing 118, and may be dependent upon the type of syringe utilized.

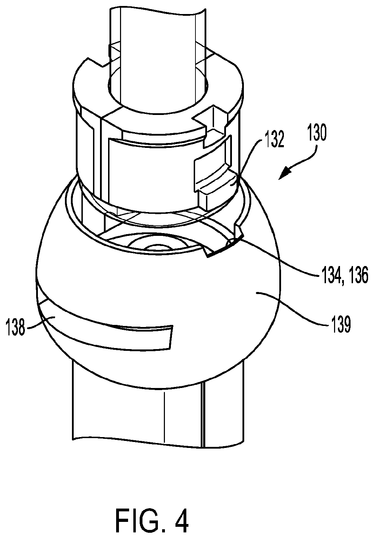

In order to couple the clips 124 along with proximal end 105 of the syringe 102 to the distal end of the housing 118, mating structures 130 are provided (see FIG. 4). While the mating structures 130 may be of any appropriate design, in the illustrated embodiment, the clips 124 are provided with outwardly extending tabs 132, while the channel 122 of the distal end 120 of the housing 118 is provided with recesses 134 adapted to receive the tabs 132. The recesses 134 include a generally axially extending portion 136, and an arcuate portion 138. In the illustrated embodiment, the arcuate portions 138 of the recesses 134 extend through the wall 139 of the housing 118. It will be appreciated, however, that in alternative embodiments, a portion or the entire length of the recesses 134 may extend through the wall 139, or be recessed, but not extending through the wall 139. In this way, movement of the tabs 132 within the axially extending portion 136 of the recesses 134 causes movement of the clips 124 and the associated syringe 102 in an axial direction within the housing 118, while movement of the tabs 132 within the arcuate portion 138 of the recesses 134 causes a rotational movement of the clips 124 and the associated syringe 102 relative to the housing 118 to lock the housing 118 with the clips 124 and syringe 102. It will be appreciated that the recesses 134 may additionally include a detent or similar structure in order to further limit movement of the tabs 132 relative to the recesses 134. Those of skill in the art will further appreciate that alternative or additional mating structures may be provided between the housing 118 and the clips 124.

To further facilitate secure coupling of the syringe 102 with the housing 118, an elastomeric washer 140 may be provided (see FIG. 1). The elastomeric washer 140 may have an appropriate cross-section, such as a round cross-section or a rectangular cross-section or an "X" shaped cross-section. The elastomeric washer 140 is assembled into the channel 122 within the housing 118 prior to assembly of the clips 124 and syringe 102 with the distal end 120 of the housing 118. In this way, the elastomeric washer 140 may assist in maintaining a secure engagement of the surfaces of the tabs 132 with the recesses 134 in the arcuate portion 138, in particular, by maintaining an axial force on the clips 124 to maintain the positions of the tabs 132 with the arcuate portions 138 of the recesses 134. It will further be appreciated that the elastomeric washer 140 may likewise allow for accommodation of variations in thickness in the flange 106 of the syringe 102.

While the coupling of the syringe 102 with the housing 118 has been described and illustrated in connection with a plurality of clips 124 disposed around a proximal end 105 of the barrel 104 and the flange 106 of syringe 102 and received in channel 122 at a distal end of the housing 118, it will be appreciated by those of skill in the art that alternative coupling arrangements may be provided. For example, a flange of a syringe may be seated against a distal end of a device housing, and one or more clips clamped around the outer surfaces of both the distal end of the housing and the flange. By way of further example, the device housing may include a transversely disposed slot, such that the flange of a barrel may be moved transversely into position relative to the barrel.

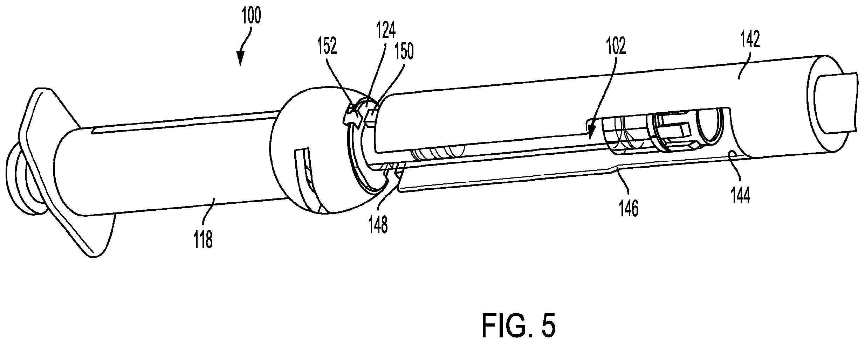

In order to assist in assembly of the syringe 102, a wrench tool 142 may be provided. As illustrated, for example, in FIG. 5, the wrench tool 142 may include an internal channel 144 sized to receive the syringe 102. While illustrated in fragmentary form, those of skill in the art will appreciate that the wrench tool 142 may include a handle that may be, for example, in axial alignment with the wrench tool 142 or disposed at an angle to the axis of the wrench tool. In order to facilitate, the attachment of the wrench tool 142 to the syringe 102, an axially extending access opening 146 may be provided, allowing the wrench tool 142 to be slid over the syringe 102 either in the radial direction, or in the axial direction. In order to facilitate assembly of the syringe 102 and coupled clips 124 with the housing 118, a distal edge 148 of the wrench tool 142 may include one or more axially extending protrusions 150 sized to be received in corresponding recesses 152 in a distal surface of the clips 124. In this way, the axially extending protrusion 150 may engage the recesses 152 and rotate the clips 124 relative to the housing 118 to couple the syringe 102 and clips 124 with the housing 118.

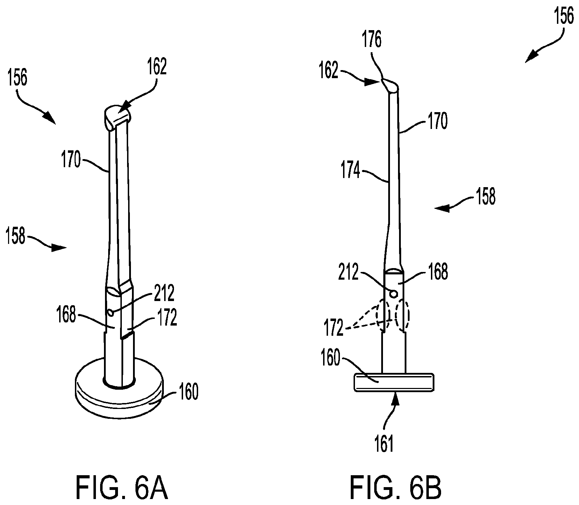

The controlled multidosing delivery device 100 further includes a drive shell 154 and a plunger rod 156. Turning first to the plunger rod 156, which is illustrated in greater detail in FIGS. 6A and 6B, the plunger rod 156 includes an elongated shaft 158 having a contact button 160 at a proximal end and a pushing feature 162 at the distal end. While the contact button 160 may have any appropriate shape, in at least one embodiment, the contact button 160 includes a concave contact surface 161, which may enhance the positioning of a user's finger against the contact surface 161. In at least one embodiment, the contact surface 161 includes texturing, which may enhance gripping by a user's finger.

In assembly, the elongated shaft 158 is received in a through hole 164 in a proximal wall 166 of the housing 118 (see FIGS. 2A and 2E). The elongated shaft 158 and the opening 164 preferably are shaped such that the plunger rod 156 is inhibited from rotating relative to the housing 118 in use. While the elongated shaft 158 may have any appropriate cross-section, a proximal portion 168 of the illustrated elongated shaft 158 has a generally rounded cross-section with segments removed along opposite sides. That is, the periphery of the shaft includes opposed arcuate sections with opposed segments removed to provide opposed chords or flats 172 between the arcuate sections. The through hole 164 of the housing 118 includes provides a similar mating structure that allows the proximal portion 168 of the elongated shaft 158 to be axially translated through the opening 164, yet preventing rotation of the elongated shaft 158 relative to the housing 118 once assembled.

Similarly, the distal portion 170 of the elongated shaft 158 may include any appropriate cross-section, so long as the elongated shaft 158 provides sufficient strength to perform its pushing function. The distal portion 170 of the illustrated embodiment includes a narrowed cross-section relative to the cross-section of the proximal portion 168. In this embodiment, the distal portion 170 includes a rectangular cross-section, although the cross-section may be other than illustrated. The pushing feature 162 at the distal end of the elongated shaft 158 protrudes from a side surface 174 of the distal portion 170 of the elongated shaft 158 to present a relatively sharp finger 176 (see FIG. 6B) for engagement with structure within the drive shell 154, as will be explained further below.

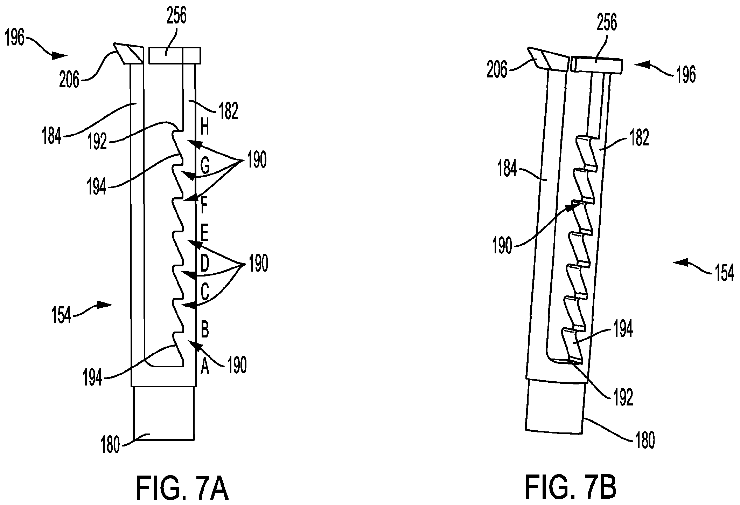

Turning now to FIGS. 7A and 7B, the drive shell 154 includes a head 180 from which extends an elongated drive arm 182 and an elongated retention arm 184. The head 180 is adapted to transmit translational motion to the plunger stopper 116 of the syringe 102 coupled to the controlled multidosing delivery device 100. The head 180 may have any appropriate cross-section. In the illustrated embodiment, the head 180 is a cylindrical structure adapted to be received through a drive opening 186 in the distal end 120 of the housing 118. As may be seen in FIGS. 2C and 2D, the opening 186 opens into the channel 122 which is adapted to receive the clips 124 and flange 106 of the syringe 102. In assembly, the elastomeric washer 140 is disposed around the periphery of the opening 186, and the flange 106 is typically seated on the elastomeric washer 140. In this way, the head 180 of the drive shell 154 is adapted to extend through the opening 186 and the elastomeric washer 140, and into a lumen of the syringe barrel 104 to drive the plunger stopper 116. While in some embodiments, the head 180 may directly engage plunger stopper 116, in the illustrated embodiment, a spacer 188 is assembled into the barrel 104 between the plunger stopper 116 and the head 180. In this way, the head 180 would engage the spacer 188, which would engage the plunger stopper 116. Those of skill in the art will appreciate that the dimensions of the head 180 and the optional spacer 188 will depend upon the structure and features of the syringe 102, for example, the volume of fluid contained in a filled syringe 102.

In order to provide translational motion to the head 180, the elongated drive arm 182 includes a plurality of forward drive engagement steps 190. The forward drive engagement steps 190 include an engagement surface 192 and a ramp 194. The engagement surface 192 faces a proximal end 196 of the drive shell 154. As may be seen in the cross-section of FIG. 8, in the assembled controlled multidosing delivery device 100, the plunger rod 156 is disposed such that when the finger 176 of the plunger rod 156 may engage an engagement surface 192 of the forward drive engagement step 190 (see also A in FIG. 7A). As a result of this engagement, axial movement of the plunger rod 156 in the distal direction results in a corresponding axial movement of the drive shell 154. This axial movement of the drive shell 154 by the plunger rod 156 moves the head 180 of the drive shell 154 to provide a corresponding movement of the spacer 188 (if utilized), and the plunger stopper 116 to cause a measured dispensing of fluid from the barrel 104. That is, the spatial frequency of these engagement surfaces 192 of the forward drive engagement steps 190 corresponds to the injection stroke for each injection volume delivered by the dispensing syringe 102. In other words, the axial distance between adjacent engagement surfaces 192 (see A-H in FIG. 7A) corresponds to the injection stroke for delivering each volume. After dispensing fluid from the syringe 102, the distal portion 170 of the plunger rod 156 flexes to allow the finger 176 of the plunger rod 156 to ride along the adjacent ramp 194 adjacent the engagement surface 192 as the plunger rod 156 moves proximally relative to the drive shell 154.

In order to ready the controlled multidosing delivery device 100 to provide subsequent injection strokes, the plunger rod 156 is biased in the proximal direction relative to the housing 118. In order to bias to plunger rod 156 away from the housing 118, a biasing structure such as spring 163 is provided between the plunger rod 156 and the housing 118. As may be seen in FIG 8, the spring 163 may be disposed about the elongated shaft 158 and disposed between the contact button 160 and the proximal wall 166 of the housing 118. In the illustrated embodiment, the proximal wall 166 of the housing 118 may include a recessed area or well 167, and a sleeve 165 may be disposed about the spring 163 and slidably disposed within the well 167. As the spring 163 bears on an inwardly facing flange 165A of the sleeve 165, the sleeve 165 will travel outward from the well 167 under the biasing force of the spring 163. It will be appreciated that, in at least some embodiments, the length of a stroke of the plunger rod 156 is limited by the lesser of the separation of the distal edge 165B of the sleeve 165 from the proximal surface 167A of the well 167, and the separation of the distal surface 160A of the contact button 160 from the proximal surface 118A of the housing 118. In at least some embodiments, however, the distal edge 165B of the sleeve 165 will not contact the proximal surface 167A of the well 167. In such an embodiment, the length of the stroke of the plunger rod 156 will be limited by the separation of the distal surface 160A of the contact button 160 from the proximal surface 118A of the housing.

Inasmuch as the plunger rod 156 is biased toward its original axial position, the finger 176 rides along the ramped surface 194 disposed adjacent the engagement surface 192 utilized for the injection, moving the finger 176 inward. The elongated shaft 158 of the plunger rod 156 outward under bias as the finger 176 reaches the next engagement surface 192 of the drive shell 154. In this engaged position, the controlled multidosing delivery device 100 and syringe 102 are ready to dispense the next measured injection of fluid from the syringe 102 upon depression of the plunger rod 156.

As is likewise apparent from FIG. 8, the drive shell 154 is disposed within an axially extending chamber 200 within the housing 118. In order to prevent proximal movement of the drive shell 154 as the drive shell 154 is advanced towards and through the distal end 120 of the housing 118, retention structure is provided between the drive shell 154 and the housing 118. It will be appreciated by those of skill in the art that preventing such proximal movement of the drive shell 154 may assist in providing accuracy in the volume of the injected dose; this may be particularly apparent when injecting viscous formulations or injecting into a high pressure chamber. In the illustrated embodiment, the housing 118 includes a sawtooth arrangement of a plurality of engagement ledges 202 and adjacent ramped surfaces 204, while the elongated retention arm 184 of the drive shell 154 includes a retention finger 206. As is illustrated in FIGS. 2A and 8, opposed sides 208, 210 of the axially extending chamber 200 of the housing 118 may include such a plurality of ledges 202.

In operation, axial movement of the plunger rod 156 and engagement of the pushing feature 162 with an engagement surface 192 of the drive shell 154 advances the drive shell 154 in the distal direction within the housing 118. As the drive shell 154 advances within the housing 118, the elongated retention arm 184 of the drive shell 154 deflects inward as the retention finger 206 rides along a ramped surface 204 of the housing 118. Inasmuch as the elongated retention arm 184 is biased to its outward, free position, as the retention finger 206 reaches the end of the ramped surface 204, the retention finger 206 moves outward to engage the next engagement ledge 202 of the housing 118 to prevent the drive shell 154 from movement in a proximal direction relative to the housing 118. In this way, the retention finger 206 advances from engagement ledge 202 to engagement ledge 202 of the sawtooth pattern with each end of dose. While the engagement ledges 202 of the illustrated embodiment are disposed at a right angle to the adjacent ramped surface 204 and the longitudinal axis of movement of the drive shell 154 within the housing 118, it will be appreciated that the angle at the defined peak may be other than as illustrated, so long as a secure engagement of the retention finger 206 is provided with the ledge 202.

The number of teeth or peaks presented between adjacent ramped surfaces 204 and engagement ledges 202 defines than the number of injections that may be administered using the controlled multidosing delivery device 100. If an initial movement of the plunger rod 156 and the drive shell 154 is utilized in a priming operation, then the number teeth or peaks is one greater than the number of injections that may be administered. Thus, in the illustrated embodiment, the controlled multidosing delivery device 100 may be utilized to dispense fluid from the associated syringe 102 eight times, or the controlled multidosing delivery device 100 may be utilized to prime the syringe 102, and to administer seven injections.

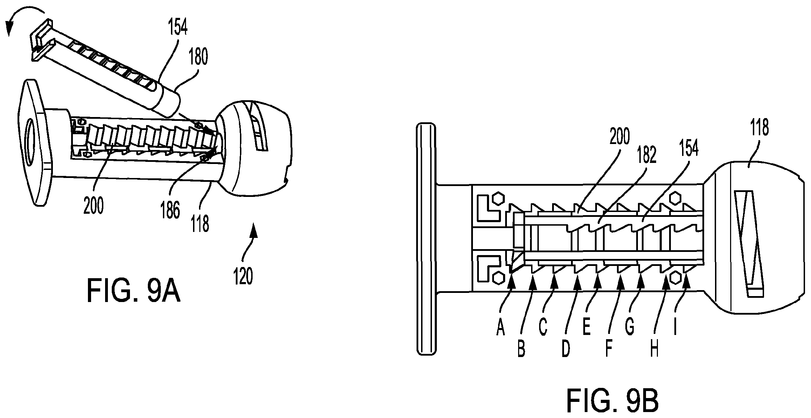

Referring to FIG. 9A, in assembly of the controlled multidosing delivery device 100, a distal end of the drive shell 154 is angled toward the housing 118 to insert the drive shell 154 into the axially extending chamber 200 of the housing 118 and slide the head 180 of the drive shell 154 through the opening 186 in the distal end 120 of the housing 186. Referring to FIGS. 1 and 8, the elongated shaft 158 of the plunger rod 156 is then inserted into the sleeve 165, and the spring 163 assembled between the sleeve 158 and the elongated shaft 158. Alternatively, the spring 163 may be assembled into the sleeve 158, and then elongated shaft 158 of the plunger rod 156 inserted into the sleeve 158/spring 163 subassembly. The elongated shaft 158 is then inserted into the opening 164 in the proximal wall 166 of the housing 118, and the elongated shaft 158 is advanced between the elongated drive arm 182 and elongated retention arm 184 of the drive shell 154 assembled into the housing 118. The plunger rod 156 is disposed in its final assembled position when the plunger rod 156 is in its fully inserted position within the drive shell 154 with the pushing feature 162 disposed toward the elongated drive arm 182 of the drive shell 154, and, more particularly, a distally disposed engagement surface 192 of the drive shell 154. In the illustrated embodiment, the plunger rod 156 is fully inserted when pushing feature 162 of the plunger rod 156 abuts the engagement surface 192 at a distal internal surface of the drive shell 154.

In order to retain the elongated shaft 158 of the plunger rod 156 in position within the housing 118, a retaining arrangement is provided. In the illustrated embodiment, the elongated shaft 158 is provided with a through hole 212, and a retaining pin 214 is inserted into the through hole 212 orthogonal to the axis of the plunger rod 156. In assembly, the plunger rod 156 is depressed slightly to provide ready access to the through hole 212, and to properly position the spring 163 before the retaining pin 214 is inserted. Inasmuch as the length of the retaining pin 214 is greater than the length of the through hole 212, the end(s) of the retaining pin 214 protruding from the through hole 212 act to retain the plunger rod 156 within the housing 118. Further, inasmuch as the effective length of the retaining pin 214 within the through hole 212 is less than the depth of the axially extending chamber 200 of the housing 118, the plunger rod 156 may be axially translated within the housing 118 between the elongated drive arm 182 and the elongated retention arm 184 of the drive shell 154.

Referring to FIG. 1, a cover 220 may be provided to overlay a portion or the entirety of the opening into chamber 200 in the housing 118. The cover 220 may be coupled to the housing 118 by any appropriate arrangement. By way of example only, the cover 220 and housing 118 may include a plurality of mating protrusions and recesses of any appropriate distribution and shape. In the illustrated embodiment, the cover 220 includes a plurality of protrusions 222 in the form of posts extending from a surface 224 of the cover 220, while the housing 118 includes a corresponding plurality of recesses 226 in the form of bores. The protrusions 222 and recesses 226 may include interlocking or engaging structures or may have disparate cross-sections in order to inhibit separation of the cover 220 from the housing 118. For example, the protrusions 222 in the form of bores may have a rounded cross-section, while the recesses 226 in the form of bores may have a hexagonal cross-section or the like in order to provide an interference fit. Those of skill in the art will appreciate, however, that the mating structures may be of alternative designs, including, for example, a hinged design or separable hinge design.

The syringe 102 may coupled to the housing 118 as explained above. It will be appreciated that operation of the controlled multidosing delivery device 100 does not require that the cover 220 be positioned on the housing 118. Moreover, the syringe 102 is not required to be coupled to the housing 118 only after the placement of the cover 220 with the housing 118. It is noted, however, that the placement of the cover 220 on the housing 118 covers and protects the operation of the internal structures of the housing 118, while maintaining a clean environment.

The housing 118 may additionally include a flange 230 positioned at the proximal end 119 of the housing 118. In at least some embodiments, the flange 230 may extend from either side of the housing 118 a sufficient distance to provide engagement surfaces for the user's fingers during operation. It will be appreciated that the flange 230 may afford the user more stability during the injection procedure. The flange 230 may also be used to aid attachment of the controlled multidosing delivery device 100 to a stereotactic frame or robotic arm. Alternatively or additionally, further structure may be provided with the housing 118 to facilitate coupling to a stereotactic frame or robotic arm.

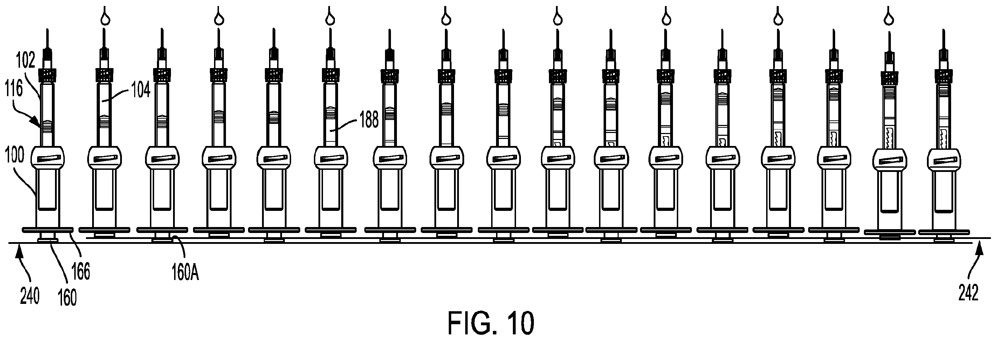

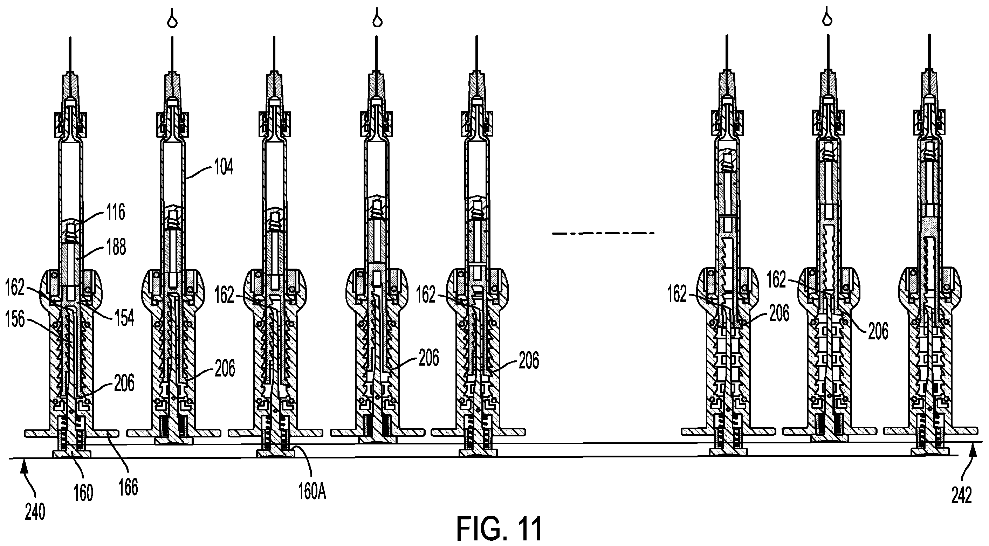

An exemplary operation of the controlled multidosing delivery device 100 and coupled syringe 102 is illustrated in FIG. 10 in a sequence of positions to deliver a plurality of sequential injections, while a cross-section of the controlled multidosing delivery device 100 and coupled syringe 102 during a corresponding series of injections is illustrated in FIG. 11. At the start of each injection, the contact button 160 is spaced from the proximal wall 166 of the housing 118 at a start of dose position (the proximal surface of the contact button 160 prior to depression being identified in FIGS. 10 and 11 by line 240). The contact button 160 is then depressed until the distal surface 160A of the contact button 160 contacts the proximal wall 166 of the housing 118, that is, the end of dose position (identified in FIGS. 10 and 11 by line 242, which illustrates the distance that the proximal surface of the contact button 160 has traveled). The difference in positions 240 and 242 is the user injection stroke 244. As the user depresses the contact button 160, axial translation of the plunger rod 156 causes axial translation of the engaged drive shell 154, which causes axial translation of the spacer 188 (if included) and the plunger stopper 116, as discussed above, to dispense a predetermined volume of fluid from the barrel 104 of the syringe 102. This dispensing is signified in FIGS. 10 and 11 as a droplet.

As the drive shell 154 is axially translated relative to the housing 118 during dispensing, the retention finger 206 of the drive shell 154 is moved against its outward bias and rides along a ramped surface 204 within the channel 122 of the housing 118 until such time as the retention finger 206 reaches an engagement ledge 202 within the channel 122. Under the force of the bias, the retention finger 206 then moves outward to engage the subsequent engagement ledge 202. This engagement between the retention finger 206 and the engagement ledge 202 acts to prevent movement of the drive shell 154 in a proximal direction relative to the housing 118. In at least some embodiments, this movement of the retention finger 206 outward and into engagement with the engagement ledge 202 provides an audible click, which may provide the user with an additional indication that the dose has been delivered.

As the contact button 160 is depressed during dose delivery, the biasing structure or spring 163 is compressed. As pressure is released from the contact button 160, the force of the spring 163 returns the contact button 160 to the start of dose position 240. As the plunger rod 156 concurrently translates axially in the proximal direction, the distal portion 170 of the elongated shaft 158 moves against a bias of the finger 176 of the pushing feature 162 into engagement with an engagement surface 192 of the drive shell 154, sliding the finger 176 along the adjacent ramp 194 until the finger 176 is again biased into contact with the next engagement surface 192. The components of the controlled multidosing delivery device 100 and coupled syringe 102 are then in position for delivery of the subsequent dose. As may be seen in FIG. 11, this process is repeated to deliver subsequent, sequential doses of fluid from the syringe 102. In view of the number of engagement surfaces 192 of the drive shell 154 and engagement ledges 202 of the housing 118, the illustrated controlled multidosing delivery device 100 and coupled syringe 102 may be utilized to deliver a total of eight doses, or a priming dose followed by seven subsequent measured doses of equal volume.

Thus, when the plunger rod 156 is sequentially depressed by the user, the retention finger 206 continues to advance with the advancement of the drive shell 154 until the retention finger 206 is advanced to a final location that is the most distally disposed engagement ledge 202 of the housing 118. Accordingly, any subsequent attempt by the user to depress the plunger rod 156 does not translate into any further advancement of the plunger stopper 116 within the barrel 104 of the syringe 102. The controlled multidosing delivery device 100 and coupled syringe 102 can then be disposed appropriately.

Referring to FIG. 2A, in order to visually provide a user with an identification of the number of doses that have been delivered, the housing 118 may be provided with a plurality of windows 250 that extend through a wall 252 of the housing 118 and into the axially extending chamber 200. In this way, a portion of the drive shell 154 may be visible through a window 250 that corresponds to the axial location of the drive shell 154 within the channel 122. For example, the proximal end of the elongated drive arm 182 and or the elongated retention arm 184 of the drive shell 154 may be visible through the windows 250 as the drive shell 154 is advanced. By way of example, the proximal end of the elongated drive arm 182 may include an extension 256 that is positioned for viewing through the windows 250 as the drive shell 154 advances. The external surface of the housing 118 may include dosage markings identifying the number of doses dispensed when the extension 256 is disposed adjacent the corresponding windows 250.

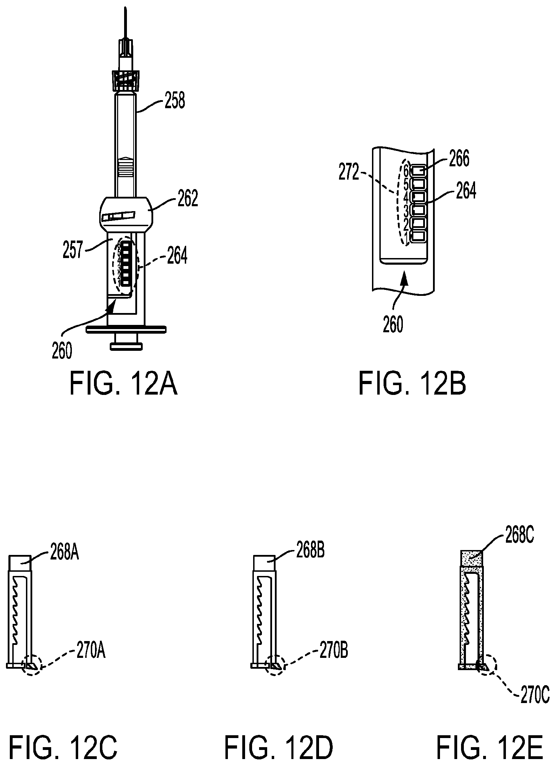

Those of skill in the art will appreciate, however, that windows may be alternatively or additionally disposed with the controlled multidosing delivery device 257 and coupled syringe 258. As illustrated in FIG. 12A, for example, a cover 260 to the housing 262 may include a visual indicator 264 of the state of the coupled syringe 258. For example, the visual indicator 264 may include one or more windows 266, the illustrated embodiment including a plurality of windows 266. The disposition of the proximal end of the drive shell 268 may be viewed through the window 266, for example, by viewing the location of the retention finger 270A-270C of the drive shell 268. As the drive shell 268 is advanced in the distal direction relative to the housing 262, the successive positions of the drive shell 268 will be visible through the window 266. In order to provide optimal visualization, the color of at least a viewed portion of the drive shell 268 may be in contrast relative to that of cover 260. A contrasting color may be achieved by either printing contrasting mark on retention finger 270B (see FIG. 12D), for example. A similar viewing result may also be achieved by manufacturing drive shell 268C in the contrasted color to the cover 260(see FIG. 12E).

Markings 272 may be provided adjacent the window(s) 266. Any appropriate marking scheme may be utilized, and the markings 272 may be applied to be viewed from the proximal or distal end of the controlled multidosing delivery device 257. For example, the markings may be designed to indicate the number of doses delivered, the number of doses left to be delivered, and/or the cumulative volume of fluid delivered or the volume of fluid remaining in the syringe 258; markings may be designed to include one or more priming steps and number of doses remaining in the syringe 258.

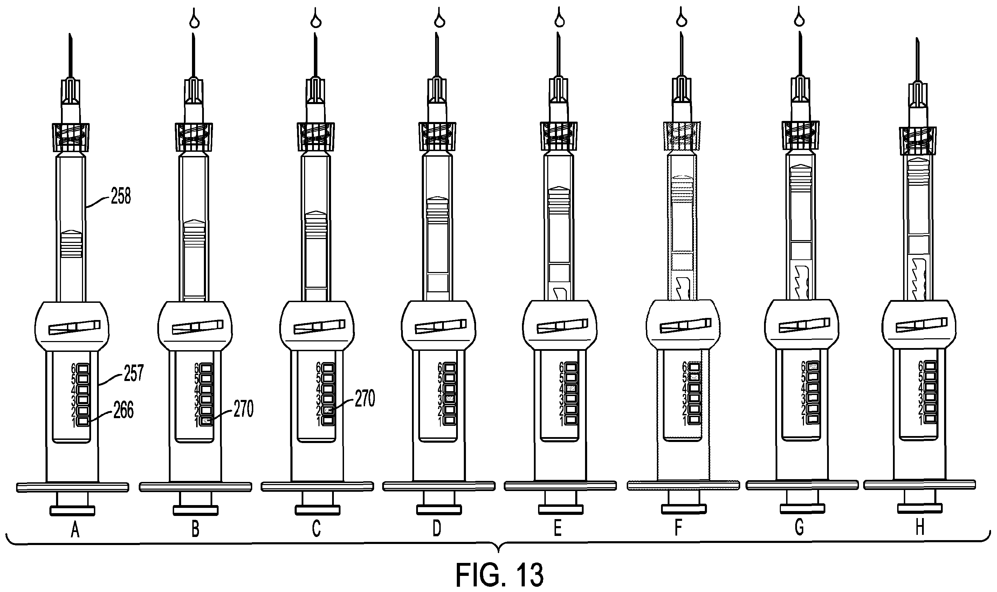

FIG. 13 illustrates the controlled multidosing delivery device 257 and coupled syringe 258 in various stages of injecting six sequential doses and corresponding indicator status. The device with attached injection needle prior to priming or injection is illustrated in A. Priming may be achieved by following the same procedure as an injecting operation. After priming, the controlled multidosing delivery device 257 and coupled syringe 258 will be disposed as illustrated in B, the retention finger 270 of the drive shell 268 being visible in the window 266 at the position identified as "1" on the cover 260. After the first injection, the retention finger 270 is visible through the window 266 at the position identified as "2" on the cover 260. With each subsequent injection, the visible position of the retention finger 270 progresses through the window 266 as the drive shell 268 advances distally (see C through G). Once the controlled multidosing delivery device 257 and coupled syringe 258 reach the position identified as "6" in the window 266 (see G), a single dose is contained in the syringe 258. Following delivery of the final dose, the retention finger 270 of the drive shell 268 is no longer visible in the window 266, indicating that all doses have been delivered (see H in FIG. 13). Visual examination of the contents of the syringe 258 provides additional confirmatory visual indication, if desired or required.

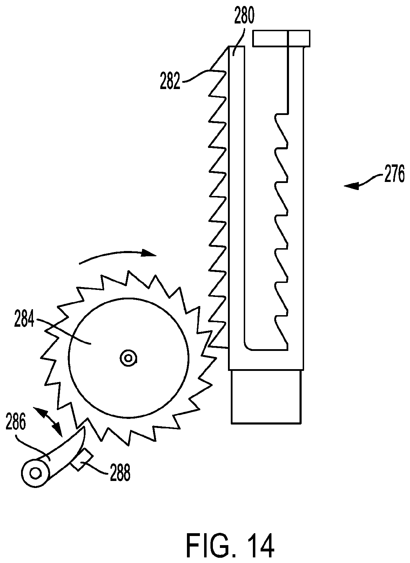

Those of skill in the art will appreciate that various elements of the structures described in detail herein may be modified while still coming within the purview of the appended claims. For example, an alternate embodiment of an arrangement for limiting proximal axial movement of a drive shell 276 within a housing is illustrated in FIG. 14. While the accompanying structures of the controlled multidosing delivery device are not illustrated in their entirety in FIG. 14, those of skill in the art will appreciate that the same or similar structures may be provided in conjunction with the elements illustrated in FIG. 14. In this embodiment, an external surface 278 of an elongated retention arm 280 of the drive shell 276 is provided with a rack 282, as opposed of an internal surface of the housing including a rack. In order to prevent movement of the drive shell 276 in a proximal direction, a sawtooth gear 284 may be a rotatably mounted relative to the housing to engage with the rack 282. The gear 284 may be rotatably mounted to allow the gear 284 to rotate as the drive shell 276 is translated axially in the distal direction with each dose administration, yet prevented from rotating when a force is asserted to attempt to move the drive shell 276 in a proximal direction. While any appropriate limiting structure may be provided, rotation in reverse direction is prevented in the illustrated embodiment by a spring-biased pawl 286 and a stop feature 288. Thus, as the drive shell 276 is advanced in the distal direction, the illustrated gear 284 may rotate in the clockwise direction to accommodate the movement of the drive shell 276, the spring-biased pawl 286 being permitted to pivot in the counterclockwise direction to accommodate the rotation of the gear 284. When rotation of the gear 284 ceases, the spring-biased pawl 286 is biased back into position against the stop feature 288 to prevent counterclockwise rotation of the gear 284. Alternatively, a pressure angle between the teeth of the rack 282 and the teeth of the gear 284 may be optimized to inhibit proximal travel of the drive shell 276.

The controlled multidosing delivery devices and syringes of this disclosure may be utilized in various applications and procedures. For example, a controlled multidosing delivery device and syringe may be utilized in single site deliveries or in multiple sites in a given tissue.

It will be appreciated that at least some of the controlled multidosing delivery devices disclosed herein may be beneficial for use in emerging technologies. For example, some injectable substances are extremely potent and can potentially pose a safety risk to the user administering the treatment. A number of emerging treatments of cancer involve the injection of a therapeutic agent locally into the tumor, for example. These agents may include oncolytic viruses, PDL-1, immunotherapeutic agents and the like, which may pose such a safety risk.

Further, the use of controlled multidosing delivery devices, such as the devices disclosed herein, may provide additional benefits by allowing dose splitting of a volume of an injectable substance into smaller fractions at different sites of a target tissue. For example, tumors typically have necrosis that inhibit distribution of a therapeutic agent, thereby limiting its effectiveness. One way to overcome this is to inject in multiple sites of the tumor to enhance bioavailability and biodistribution of the therapeutic agent. Further, inasmuch as some cancer therapeutics that are designed stimulate the patient's immune system to fight cancer cells, providing multiple injections on the periphery of the cancerous lesion may provide therapeutic benefits through an enhances immune response to the tumor. The dose splitting provided by at least some of the controlled multidosing delivery devices disclosed herein may provide improved distribution of the delivered agent throughout a tumor, for example. Also, reducing the volume of each dose administered by splitting the total dose to be administer into smaller volumes may minimize the risk of drug leakage (extravasation) from the point of injection. Such multiple dosing delivery systems also may allow the clinician to focus on the anatomical aspect of the injection without diversion of clinician's cognitive faculties to the mathematical aspect of injection volume calculation. The use of a single controlled multidosing delivery device and syringe to administer injections into tumors at multiple locations for a given patient may allow the clinician to employ the same injection needle without the need for priming between injections.

Additionally, the volume of therapeutic agent is typically metered based on the size of the tumor. For example, in case of skin carcinomas, there may be multiple lesions (same and/or different size) to be treated. A device such as the controlled multidosing delivery devices disclosed, which enabling tracking of fractional doses, may alleviate the burden on the clinician to ensure that the therapeutic agent is metered accurately and appropriately.

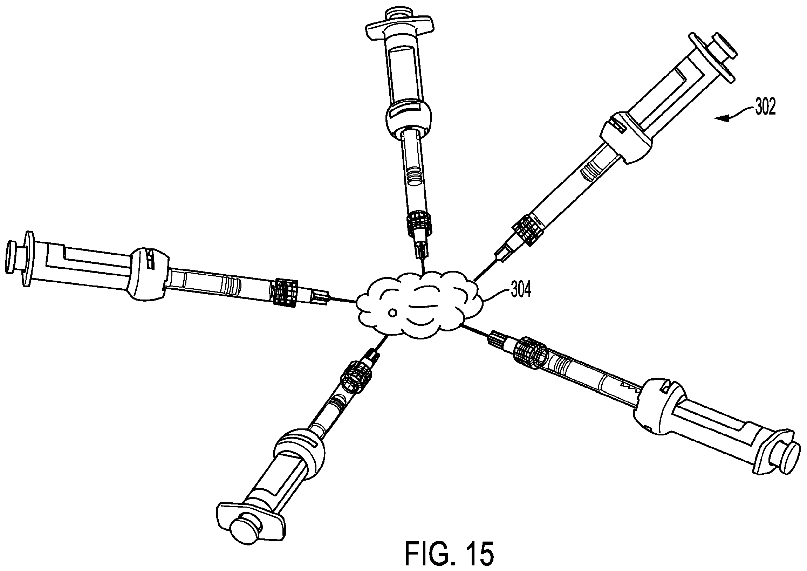

One such example of the utilization of a controlled multidosing delivery device and syringe (collectively identified as 302) in the administration of therapeutic agents in solid tumors is illustrated in FIG. 15. The controlled multidosing delivery device and syringe 302 with injection needle may be utilized to inject an anti-cancer therapeutic agent at several locations of the tumor 304. In the illustrated arrangement, the controlled multidosing delivery device and syringe 302 is utilized to inject five (5) doses of the therapeutic agents at five (5) sites selected by the clinician. The clinician can focus exclusively on proper injection site selection while delegating metering of correct dose to controlled multidosing delivery device and syringe 302.

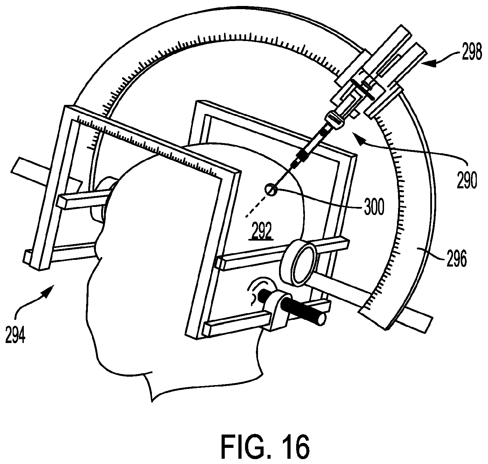

At least some of the controlled multidosing delivery devices and syringes may be utilized to deliver a plurality of doses from a single insertion of a cannula. For example, a controlled multidosing delivery device and syringe, such as the arrangements disclosed herein, may be utilized in providing multiple injections in the brain, as illustrated, for example, in FIG. 16. In use, the syringe is filled with an injectable solution, and the controlled multidosing delivery device is attached to the filled syringe. The controlled multidosing delivery device and syringe are then primed. The primed controlled multidosing delivery device and syringe (collectively identified as 290) may be maintained in a desired position relative to the skull 292 of a patient by any appropriate coupling framework 294. In the illustrated embodiment, a stereotactic frame 296 mounted with the coupling framework 294. The primed controlled multidosing delivery device and syringe 290 is coupled to the stereotactic frame 296 by a single-axis micromanipulator 298.

Secured with the single-axis micromanipulator 298, the controlled multidosing delivery device and syringe 290 may be advanced only along the axis of the controlled multidosing delivery device and syringe 290, the axis being aligned with a delivery cannula of the controlled multidosing delivery device and syringe 290. The delivery cannula of the controlled multidosing delivery device and syringe 290 may then be aligned with an opening 300 in the skull 292.

Coarse manipulation of the controlled multidosing delivery device and syringe 290 may be utilized to drive the delivery cannula through the brain's meninges close to the most distal target injection site or the cannula may be insert through an incision through the brain's meninges. Once close, fine manipulation allows the clinician to dispose the tip of the delivery cannula to the predetermined injection site. The controlled multidosing delivery device and syringe 290 is utilized to deliver a first dose of the injectable solution to the predetermined injection site. The controlled multidosing delivery device and syringe 290 is moved in a retraction direction to through fine manipulation using micromanipulator to retrace the delivery cannula entry path to position the delivery cannula in the second predetermined injection site. The controlled multidosing delivery device and syringe 290 is actuated to deliver second dose through the delivery cannula to the second predetermined injection site. This process may be repeated several times as the treatment modality would require subject to the availability of injectable fluid in the controlled multidosing delivery device and syringe 290. Ultimately, the cannula is pulled out from the tissue with minimum surgical trauma to the tissue. In this way, a controlled multidosing delivery device and syringe 290 such as devices of this disclosure may be utilized to deliver a controlled volumes of an injectable solution at different depth of a site. Significantly, multiple, equal volumes may be injected at different depths via a single insertion of the delivery cannula. The delivery cannula may be rigid or flexible (like a catheter, for example), and may have a blunt or sharp tip.

It will be appreciated that one or more imaging modality may be utilized as guidance in the positioning of the delivery cannula in the brain. The utilization of a multidosing delivery device may facilitate the accurate administration of small doses of injectable fluid, such as, for example, volumes of 100 microliters or less. Several applications involving injections of cells are likely to involve sub-milliliter (microliter) injection volumes. Because of sedimentation problems with cells, treatments involving injection of nerve cells, stem cells and the like need to be concentrated. In this way, different strengths of treatment involving cells may be achieved by varying the injection volume, which would be in the order of microliters. Those of skill in the art will appreciate that the utilization of a controlled multidosing delivery device and syringe in the injection of therapeutic agent into a brain, may assist in minimizing complications from the procedure by minimizing the number of insertions through the meninges, and also help administer accurate, precise doses. Inasmuch as brain tumors are typically diffuse, treatment may be benefited from injections in more than one location. Injecting in more than one location may additionally benefit some other brain treatment applications involving injection of a fluid that includes cells (e.g., stem cells) in the brain, particularly inasmuch as cells deposited in the proximity of target injection site and may still have potential therapeutic benefit. Also, distributing cells over several locations rather than injecting all of them in one location may potentially improve nutrient flow to these cells, thereby increasing time for which the cells are viable.

As will be understood by those of skill in the art, the components of the disclosed controlled multidosing delivery devices and syringes according to this disclosure may be made by any appropriate method. For example, the drive shell, housing, cover, plunger rod and sleeve may be injection molded or 3D printed, or the like.

It will readily be understood by one having ordinary skill in the relevant art that the disclosed invention has broad utility and application. Those of skill in the art will appreciate that at least some of the controlled multidosing delivery devices according to this disclosure facilitate multi-dosing from a syringe, including multi-dosing from a prefilled syringe. In at least some of the disclosed controlled multidosing delivery devices, user's start of injection position is constant relative to the point of injection in that the contact button is returned to an original, readied position following each injection.

Further, while the structure of controlled multidosing delivery devices discussed in detail in this disclosure have been designed to delivery eight doses (or seven doses and priming), alternative embodiments consistent with the teachings of this disclosure may be structured to deliver multiple doses, but in a greater or lesser number by varying the number of forward engagement surfaces of the drive shell and the number of retention surfaces. For example, alternative embodiments may provide the delivery of two, three, four, five, six, seven, nine or more doses by providing two, three, four, five, six, seven, nine or more forward engagement surfaces of the drive shell and the two, three, four, five, six, seven, nine or more retention surfaces, respectively.

In at least some embodiments of the controlled multidosing delivery device, the stereotactic reference for start and end of fractional dose administration remains the same irrespective of dose fraction sequence index. This may allow the clinician use to focus only the injection target, and may alleviate at least a portion of the cognitive burden on the user clinician to track and/or calculate the start and end of dose delivery position.

In at least some embodiments of the controlled multidosing delivery device, the available length of travel of the plunger rod may facilitate delivery of an accurate, precise milliliter or microliter fractional dose. At least some embodiments of the controlled multidosing delivery device allow for the delivery of equal volumes of available injectable substance from a syringe.

At least some embodiments of the controlled multidosing delivery device facilitate successive delivery of a fraction of the volume of injectable substance in an associated syringe without the need for priming after initial priming. In at least some embodiments, the controlled multidosing delivery device may inhibit movement of a plunger stopper of a syringe in a proximal direction as a result of back pressure originating at a patient end.

At least some embodiments of the controlled multidosing delivery device provide a visual indication of one or more of the number of doses administered and/or remaining, and/or the volume of injectable substance administered or remaining in the associated syringe.

The controlled multidosing delivery devices may be utilized with prefilled syringes, with syringes wherein the user can draw the injectable fluid from a vial, and with combinations thereof.

Any embodiment discussed and identified as being "preferred" should be considered to be part of a best mode envisioned to illustrate the present invention. Other additional embodiments are also discussed to exemplify and illustrate variations within the scope of the disclosed invention. Moreover, adaptations, variations, modifications, and equivalent arrangements, will also be implicitly disclosed by the embodiments described herein and would also fall within the scope of the present invention disclosed herein.

It will be appreciated that the foregoing description provides examples of the disclosed system and technique. However, it is contemplated that other implementations of the disclosure may differ in detail from the foregoing examples. All references to the disclosure or examples thereof are intended to reference the particular example being discussed at that point and are not intended to imply any limitation as to the scope of the disclosure more generally. All language of distinction and disparagement with respect to certain features is intended to indicate a lack of preference for those features, but not to exclude such from the scope of the disclosure entirely unless otherwise indicated.

Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context.

Accordingly, this disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

## Claims

1. A controlled multidosing delivery device (100) for use with a syringe (102) including a barrel (104), plunger stopper (116), and a delivery conduit (108), the controlled multidosing delivery device (100) comprising:
a housing (118) defining an axis and including a proximal end (119), a distal end (120), an axially extending chamber (200) including a first opening to the proximal end (119) of the housing (118) and a second opening to the distal end (120) of the housing (118), the distal end (120) of the housing (118) being adapted for attachment to the syringe barrel (104) along said axis, the housing (118) being coaxial with the syringe barrel (104);
a plunger rod (156) including an elongated shaft (158) having a proximal end and a distal end, a contact button (160) disposed at the proximal end of the elongated shaft (158), and a pushing feature (162) disposed at the distal end of the elongated shaft (158), a portion of the elongated shaft (158) being disposed within the axially extending chamber (200) of the housing (118) along the axis, the proximal end of the elongated shaft (158) extending at least partially from the proximal end (119) of the housing (118), the contact button (160) being disposed external to the housing (118);
**characterized in that** the controlled multidosing delivery device (100) further comprises:
a biasing structure (163) disposed to bias the contact button (160) away from the housing (118);
a retaining structure (212, 214) adapted to inhibit removal of the plunger rod (156) and permit the plunger rod (156) to translate axially a predetermined distance;
a drive shell (154) disposed within the axially extending chamber (200) of the housing (118), the drive shell (154) including a head (180) disposed for translation along the axis to confront the stopper plunger (116) of the syringe (102), an elongated drive arm (182) and an elongated retention arm (184), the elongated drive arm (182) including a plurality of engagement surfaces (192), the plunger rod (156) being disposed between the elongated drive arm (182) and the elongated retention arm (184), the pushing feature (162) of the plunger rod (156) being biased toward at least one of the engagement surfaces (192), the pushing feature (162) being disposed to engage the at least one of the engagement surfaces (192) to translate the drive shell (154) in a distal direction;
a retention feature (202, 206) including at least one retention finger (206) and a plurality of retention surfaces (202), the retention feature being adapted to inhibit proximal movement of the drive shell (154) within the axially extending chamber (200) when retention finger (206) is engaged with at least one of the plurality of retention surfaces (202), at least one of the at least one retention finger (206) and the plurality of retention surfaces (202) being associated with the elongated retention arm (184) of the drive shell (154), the other of the retention finger (206) and the plurality of retention surfaces (202) being associated with the housing (118),
whereby, depression of the contact button (160) axially translates the elongated shaft (158) and pushing feature (162) in engagement with the at least one of the engagement surfaces (192) in a distal direction along the axis to translate the drive shell (154) in the distal direction based upon said predetermined distance to cause corresponding movement of the plunger stopper (116) within the barrel (104) for a dose delivery,
whereby the at least one retention finger (206) engages with at least one of the plurality of retention surfaces (202) to maintain an axial position of the drive shell (154) in the housing (118) following movement of the drive shell (154) in the distal direction, and
whereby the biasing structure (163) translates the plunger rod (156) in the proximal direction following translation of the drive shell (154).

2. The controlled multidosing delivery device (100) of claim 1 wherein at least one of the plurality of retention surfaces (202) and the plurality of engagement surfaces (192) forms a sawtooth configuration with a plurality of ramped surfaces.

3. The controlled multidosing delivery device (100) of any of claims 1 and 2 wherein the retention arm (184) includes the at least one retention finger (206), and the housing (118) includes the plurality of retention surfaces (202).

4. The controlled multidosing delivery device (100) of claim 1 including a gear (284) rotatably coupled to the housing (118), the gear (284) including the at least one retention finger (206).

5. The controlled multidosing delivery device (100) of any of claims 1-4 wherein the plurality of engagement surfaces forms a sawtooth configuration with a plurality of ramps, and optionally wherein the pushing feature is adapted to ride along the at least one of the plurality of ramps following the dose delivery.

6. The controlled multidosing delivery device of any of claims 1-5 wherein the retaining structure (212, 214) includes a retaining pin (214) secured with and extending from the elongated shaft (158), the retaining pin (214) being disposed within the housing (118).

7. The controlled multidosing delivery device (100) of any of claims 1-6 wherein the biasing structure (163) includes a spring (163), and optionally wherein the spring is disposed about the elongated shaft between the contact button and the proximal end of the housing, and optionally further including a sleeve disposed about the spring.

8. The controlled multidosing delivery device (100) of any of claims 1-7 wherein at least a portion of the elongated shaft (158) includes a cross-section and the first opening of the housing (118) have corresponding cross-sections such that the elongated shaft (158) may translate through the first opening but will not rotate within the first opening.

9. The controlled multidosing delivery device (100) of any of claim 1-8 wherein the syringe (102) includes a syringe proximal end (105), the controlled multidosing delivery device (100) further including at least one clip (124) adapted to couple the syringe proximal end (105) to the distal end (120) of the housing (118), and optionally wherein the syringe proximal end includes an outwardly extending flange, the at least one clip being adapted to couple the outwardly extending flange to the distal end of the housing, and optionally wherein the housing includes a channel opening to the distal end of the housing, the second opening of the housing opening into the channel, the channel being adapted to receive the syringe proximal end.

10. The controlled multidosing delivery device (100) of any of claims 1-9 wherein and initial engagement of the at least one retention finger (206) with at least one of the plurality of retention surfaces (202) provides at least one of a tactile feedback and audible click.

11. The controlled multidosing delivery device (100) of any of claims 1-10 adapted to deliver a controlled dose with each depression of the contact button (160), the drive shell (154) including at least two engagement surfaces (192), and the retention feature (202, 206) including at least two retention surfaces (202) such that the controlled multidosing delivery device is adapted to dispense at least two sequential doses.

12. The controlled multidosing delivery device (100) of any of claims 1-11 further including a visual indicator (264) of at least one of a number of doses delivered or a number of doses remaining in the syringe (102).

## Patentansprüche

1. Kontrollierte Mehrfachdosierungsvorrichtung (100) zur Verwendung mit einer Spritze (102), die einen Zylinder (104), einen Kolbenstopfen (116) und eine Abgabeleitung (108) einschließt, wobei die kontrollierte Mehrfachdosierungsvorrichtung (100) umfasst:
ein Gehäuse (118), das eine Achse definiert und ein proximales Ende (119), ein distales Ende (120), eine axial verlaufende Kammer (200) einschließt, die eine erste Öffnung zum proximalen Ende (119) des Gehäuses (118) und eine zweite Öffnung zum distalen Ende (120) des Gehäuses (118) einschließt, wobei das distale Ende (120) des Gehäuses (118) zur Befestigung am Spritzenzylinder (104) entlang der Achse geeignet ist, wobei das Gehäuse (118) koaxial mit dem Spritzenzylinder (104) ist;
eine Kolbenstange (156), die einen länglichen Schaft (158), der ein proximales Ende und ein distales Ende aufweist, einen Kontaktknopf (160), der am proximalen Ende des länglichen Schafts (158) angeordnet ist, und ein Schiebemerkmal (162), das am distalen Ende des länglichen Schafts (158) angeordnet ist, einschließt, wobei ein Abschnitt des länglichen Schafts (158) innerhalb der axial verlaufenden Kammer (200) des Gehäuses (118) entlang der Achse angeordnet ist, wobei sich das proximale Ende des länglichen Schafts (158) mindestens teilweise aus dem proximalen Ende (119) des Gehäuses (118) erstreckt, wobei der Kontaktknopf (160) außerhalb des Gehäuses (118) angeordnet ist;
**dadurch gekennzeichnet, dass** die kontrollierte Mehrfachdosierungsvorrichtung (100) weiter umfasst:
eine Vorspannstruktur (163), die dafür angeordnet ist, den Kontaktknopf (160) vom Gehäuse (118) weg vorzuspannen;
eine Haltestruktur (212, 214), die geeignet ist, um Entfernen der Kolbenstange (156) zu hemmen und zu ermöglichen, dass sich die Kolbenstange (156) axial um eine vorgegebene Strecke verschiebt;
eine Antriebshülse (154), die innerhalb der axial verlaufenden Kammer (200) des Gehäuses (118) angeordnet ist, wobei die Antriebshülse (154) einen Kopf (180), der zu Verschiebung entlang der Achse angeordnet ist, um dem Stopfenkolben (116) der Spritze (102) gegenüberzustehen, einen länglichen Antriebsarm (182) und einen länglichen Haltearm (184) einschließt, wobei der längliche Antriebsarm (182) eine Vielzahl von Eingriffsflächen (192) einschließt, wobei die Kolbenstange (156) zwischen dem länglichen Antriebsarm (182) und dem länglichen Haltearm (184) angeordnet ist, wobei das Schiebemerkmal (162) der Kolbenstange (156) in Richtung mindestens einer der Eingriffsflächen (192) vorgespannt ist, wobei das Schiebemerkmal (162) so angeordnet ist, dass es die mindestens eine der Eingriffsflächen (192) in Eingriff nimmt, um die Antriebshülse (154) in einer distalen Richtung zu verschieben;
ein Haltemerkmal (202, 206), das mindestens einen Haltefinger (206) und eine Vielzahl von Halteflächen (202) einschließt, wobei das Haltemerkmal geeignet ist, die proximale Bewegung der Antriebshülse (154) innerhalb der axial verlaufenden Kammer (200) zu hemmen, wenn der Haltefinger (206) mit mindestens einer von der Vielzahl von Halteflächen (202) in Eingriff ist, wobei mindestens eines von dem mindestens einen Haltefinger (206) und der Vielzahl von Halteflächen (202) mit dem länglichen Haltearm (184) der Antriebshülse (154) verknüpft ist, wobei das andere von dem Haltefinger (206) und der Vielzahl von Halteflächen (202) mit dem Gehäuse (118) verknüpft ist,
wodurch Niederdrücken des Kontaktknopfes (160) die längliche Welle (158) und das Schiebemerkmal (162) in Eingriff mit mindestens einer der Eingriffsflächen (192) in einer distalen Richtung entlang der Achse axial verschiebt, um die Antriebshülse (154) in der distalen Richtung auf Grundlage der vorgegebenen Strecke zu verschieben, um eine entsprechende Bewegung des Kolbenstopfens (116) innerhalb des Zylinders (104) für eine Dosisabgabe zu bewirken,
wodurch der mindestens eine Haltefinger (206) mit mindestens einer von der Vielzahl von Halteflächen (202) in Eingriff kommt, um eine axiale Position der Antriebshülse (154) im Gehäuse (118) anschließend an die Bewegung der Antriebshülse (154) in der distalen Richtung aufrechtzuerhalten, und
wodurch die Vorspannstruktur (163) die Kolbenstange (156) in der proximalen Richtung anschließend an die Verschiebung der Antriebshülse (154) verschiebt.

2. Kontrollierte Mehrfachdosierungsvorrichtung (100) nach Anspruch 1, wobei mindestens eine von der Vielzahl von Halteflächen (202) und der Vielzahl von Eingriffsflächen (192) eine Sägezahnkonfiguration mit einer Vielzahl von rampenförmigen Flächen bildet.

3. Kontrollierte Mehrfachdosierungsvorrichtung (100) nach einem der Ansprüche 1 und 2, wobei der Haltearm (184) den mindestens einen Haltefinger (206) einschließt und das Gehäuse (118) die Vielzahl von Halteflächen (202) einschließt.

4. Kontrollierte Mehrfachdosierungsvorrichtung (100) nach Anspruch 1, die ein drehbar mit dem Gehäuse (118) verbundenes Zahnrad (284) einschließt, wobei das Zahnrad (284) den mindestens einen Haltefinger (206) einschließt.

5. Kontrollierte Mehrfachdosierungsvorrichtung (100) nach einem der Ansprüche 1-4, wobei die Vielzahl von Eingriffsflächen eine Sägezahnkonfiguration mit einer Vielzahl von Rampen bildet und wahlweise wobei das Schiebemerkmal geeignet ist, um anschließend an die Dosisabgabe entlang mindestens einer von der Vielzahl von Rampen zu gleiten.

6. Kontrollierte Mehrfachdosierungsvorrichtung nach einem der Ansprüche 1-5, wobei die Haltestruktur (212, 214) einen Haltestift (214) einschließt, der an dem länglichen Schaft (158) befestigt ist und sich von diesem erstreckt, wobei der Haltestift (214) innerhalb des Gehäuses (118) angeordnet ist.

7. Kontrollierte Mehrfachdosierungsvorrichtung (100) nach einem der Ansprüche 1-6, wobei die Vorspannstruktur (163) eine Feder (163) einschließt und wahlweise wobei die Feder um den länglichen Schaft zwischen dem Kontaktknopf und dem proximalen Ende des Gehäuses angeordnet ist, und die wahlweise weiter eine um die Feder angeordnete Hülse einschließt.

8. Kontrollierte Mehrfachdosierungsvorrichtung (100) nach einem der Ansprüche 1-7, wobei mindestens ein Abschnitt des länglichen Schafts (158) einen Querschnitt einschließt und die erste Öffnung des Gehäuses (118) entsprechende Querschnitte aufweist, sodass sich der längliche Schaft (158) durch die erste Öffnung verschieben kann, sich aber nicht innerhalb der ersten Öffnung drehen wird.

9. Kontrollierte Mehrfachdosierungsvorrichtung (100) nach einem der Ansprüche 1-8, wobei die Spritze (102) ein proximales Spritzenende (105) einschließt, die kontrollierte Mehrfachdosierungsvorrichtung (100) weiter mindestens eine Klemme (124) einschließt, die geeignet ist, das proximale Spritzenende (105) mit dem distalen Ende (120) des Gehäuses (118) zu verbinden, und wahlweise wobei das proximale Spritzenende einen nach außen verlaufenden Flansch einschließt, wobei die mindestens eine Klemme angepasst ist, um den nach außen verlaufenden Flansch mit dem distalen Ende des Gehäuses zu verbinden, und wahlweise wobei das Gehäuse eine Kanalöffnung zu dem distalen Ende des Gehäuses einschließt, wobei sich die zweite Öffnung des Gehäuses in den Kanal öffnet, wobei der Kanal angepasst ist, um das proximale Spritzenende aufzunehmen.

10. Kontrollierte Mehrfachdosierungsvorrichtung (100) nach einem der Ansprüche 1-9, wobei ein anfänglicher Eingriff des mindestens einen Haltefingers (206) mit mindestens einer von der Vielzahl von Halteflächen (202) mindestens eines von einer taktilen Rückmeldung und einem hörbaren Klicken bereitstellt.

11. Kontrollierte Mehrfachdosierungsvorrichtung (100) nach einem der Ansprüche 1-10, die geeignet ist, um bei jedem Niederdrücken des Kontaktknopfes (160) eine kontrollierte Dosis abzugeben, wobei die Antriebshülse (154) mindestens zwei Eingriffsflächen (192) einschließt und das Haltemerkmal (202, 206) mindestens zwei Halteflächen (202) einschließt, sodass die kontrollierte Mehrfachdosierungsvorrichtung geeignet ist, um mindestens zwei aufeinanderfolgende Dosen abzugeben.

12. Kontrollierte Mehrfachdosierungsvorrichtung (100) nach einem der Ansprüche 1-11, die weiter einen optischen Indikator (264) mindestens einer von einer Anzahl von abgegebenen Dosen oder einer Anzahl von in der Spritze (102) verbleibenden Dosen einschließt.

## Revendications

1. Dispositif d'administration de doses multiples contrôlées (100) destiné à être utilisé avec une seringue (102) incluant un corps (104), un bouchon de piston (116) et un conduit d'administration (108), le dispositif d'administration de doses multiples contrôlées (100) comprenant :
un boîtier (118) définissant un axe et incluant une extrémité proximale (119), une extrémité distale (120), une chambre s'étendant axialement (200) incluant une première ouverture vers l'extrémité proximale (119) du boîtier (118) et une deuxième ouverture vers l'extrémité distale (120) du boîtier (118), l'extrémité distale (120) du boîtier (118) étant adaptée pour la fixation au corps de la seringue (104) le long dudit axe, le boîtier (118) étant coaxial avec le corps de la seringue (104) ;
une tige de piston (156) incluant un arbre allongé (158) présentant une extrémité proximale et une extrémité distale, un bouton de contact (160) disposé à l'extrémité proximale de l'arbre allongé (158), et un élément de poussée (162) disposé à l'extrémité distale de l'arbre allongé (158), une partie de l'arbre allongé (158) étant disposée à l'intérieur de la chambre s'étendant axialement (200) du boîtier (118) le long de l'axe, l'extrémité proximale de l'arbre allongé (158) s'étendant au moins partiellement à partir de l'extrémité proximale (119) du boîtier (118), le bouton de contact (160) étant disposé à l'extérieur du boîtier (118) ;
**caractérisé en ce que** le dispositif d'administration de doses multiples contrôlées (100) comprend en outre :
une structure d'inclinaison (163) disposée à éloigner le bouton de contact (160) du boîtier (118) ;
une structure de retenue (212, 214) adaptée pour empêcher le retrait de la tige du piston (156) et permettre à la tige du piston (156) de se déplacer axialement sur une distance prédéterminée ;
une coque d'entraînement (154) disposée dans la chambre s'étendant axialement (200) du boîtier (118), la coque d'entraînement (154) incluant une tête (180) disposée de façon à se déplacer le long de l'axe afin de faire face au piston d'arrêt (116) de la seringue (102), un bras d'entraînement allongé (182) et un bras de retenue allongé (184), le bras d'entraînement allongé (182) incluant une pluralité de surfaces de mise en prise (192), la tige du piston (156) étant disposée entre le bras d'entraînement allongé (182) et le bras de retenue allongé (184), l'élément de poussée (162) de la tige du piston (156) étant orienté vers au moins une des surfaces de mise en prise (192), l'élément de poussée (162) étant disposé de façon à se mettre en prise avec au moins une des surfaces de mise en prise (192) afin de déplacer la coque d'entraînement (154) dans une direction distale ;
un dispositif de retenue (202, 206) incluant au moins un doigt de retenue (206) et une pluralité de surfaces de retenue (202), le dispositif de retenue étant adapté pour empêcher le mouvement proximal de la coque d'entraînement (154) à l'intérieur de la chambre s'étendant axialement (200) lorsque le doigt de retenue (206) est mis en prise avec au moins une des multiples surfaces de retenue (202), au moins un parmi le ou les doigts de retenue (206) et la pluralité de surfaces de retenue (202) étant associées au bras de retenue allongé (184) de la coque d'entraînement (154), l'autre doigt de retenue (206) et la pluralité de surfaces de retenue (202) étant associé au boîtier (118),
selon lequel, la pression sur le bouton de contact (160) déplace axialement l'arbre allongé (158) et l'élément de poussée (162) en contact avec au moins une des surfaces de mise en prise (192) dans une direction distale le long de l'axe pour déplacer la coque d'entraînement (154) dans la direction distale en fonction de ladite distance prédéterminée pour provoquer un mouvement correspondant du butoir du piston (116) à l'intérieur du corps (104) pour une administration de dose,
selon lequel au moins un doigt de retenue (206) se met en prise avec au moins une des multiples surfaces de retenue (202) pour maintenir une position axiale de la coque d'entraînement (154) dans le boîtier (118) suite au déplacement de la coque d'entraînement (154) dans la direction distale, et
selon lequel la structure d'inclinaison (163) déplace la tige du piston (156) dans la direction proximale suite à la translation de la coque d'entraînement (154).

2. Dispositif d'administration de doses multiples contrôlées (100) selon la revendication 1, dans lequel au moins une de la pluralité de surfaces de retenue (202) et de la pluralité de surfaces de mise en prise (192) forme une configuration en dents de scie avec une pluralité de surfaces en rampe.

3. Dispositif d'administration de doses multiples contrôlées (100) selon l'une quelconque des revendications 1 et 2, dans lequel le bras de retenue (184) inclut au moins un doigt de retenue (206), et le boîtier (118) inclut la pluralité de surfaces de retenue (202).

4. Dispositif d'administration de doses multiples contrôlées (100) selon la revendication 1, incluant un engrenage (284) couplé de manière rotative au boîtier (118), l'engrenage (284) incluant l'au moins un doigt de retenue (206).

5. Dispositif d'administration de doses multiples contrôlées (100) selon l'une quelconque des revendications 1 à 4, dans lequel la pluralité de surfaces de mise en prise forme une configuration en dents de scie avec une pluralité de rampes, et en option dans lequel l'élément de poussée est adapté pour se déplacer le long d'au moins une de la pluralité de rampes après l'administration de la dose.

6. Dispositif d'administration de doses multiples contrôlées selon l'une quelconque des revendications 1 à 5, dans lequel la structure de retenue (212, 214) inclut une goupille de retenue (214) fixée à l'arbre allongé (158) et s'étendant à partir de celui-ci, la goupille de retenue (214) étant disposée à l'intérieur du boîtier (118).

7. Dispositif d'administration de doses multiples contrôlées (100) selon l'une quelconque des revendications 1 à 6, dans lequel la structure de rappel (163) inclut un ressort (163), et en option dans lequel le ressort est disposé autour de l'arbre allongé entre le bouton de contact et l'extrémité proximale du boîtier, et en option incluant en outre un manchon disposé autour du ressort.

8. Dispositif de distribution de doses multiples contrôlées (100) selon l'une quelconque des revendications 1 à 7, dans lequel au moins une partie de la tige allongée (158) inclut une section transversale et la première ouverture du boîtier (118) présente des sections transversales correspondantes de sorte que la tige allongée (158) puisse se déplacer à travers la première ouverture mais ne tourne pas à l'intérieur de la première ouverture.

9. Dispositif d'administration de doses multiples contrôlées (100) selon l'une quelconque des revendications 1 à 8, dans lequel la seringue (102) inclut une extrémité proximale (105), le dispositif d'administration de doses multiples contrôlées (100) incluant en outre au moins un clip (124) adapté pour coupler l'extrémité proximale (105) de la seringue à l'extrémité distale (120) du boîtier (118), et en option dans lequel l'extrémité proximale de la seringue inclut une bride s'étendant vers l'extérieur, l'au moins un clip étant adapté pour coupler la bride s'étendant vers l'extérieur à l'extrémité distale du boîtier, et en option dans lequel le boîtier inclut une ouverture de canal vers l'extrémité distale du boîtier, la deuxième ouverture du boîtier s'ouvrant dans le canal, le canal étant adapté pour recevoir l'extrémité proximale de la seringue.

10. Dispositif d'administration de doses multiples contrôlées (100) selon l'une quelconque des revendications 1 à 9 dans lequel la mise en prise initiale du au moins un doigt de retenue (206) avec au moins une de la pluralité de surfaces de retenue (202) fournit au moins un parmi un retour tactile et un clic audible.

11. Dispositif d'administration de doses multiples contrôlées (100) selon l'une quelconque des revendications 1 à 10 adapté pour délivrer une dose contrôlée à chaque pression sur le bouton de contact (160), la coque d'entraînement (154) incluant au moins deux surfaces de mise en prise (192) et l'élément de retenue (202, 206) incluant au moins deux surfaces de retenue (202) de sorte que le dispositif d'administration de doses multiples contrôlées soit adapté pour distribuer au moins deux doses séquentielles.

12. Dispositif d'administration de doses multiples contrôlées (100) selon l'une quelconque des revendications 1 à 11, incluant en outre un indicateur visuel (264) d'au moins une d'un nombre de doses administrées ou d'un nombre de doses restant dans la seringue (102).
